# EUROPÄISCHE PATENTSCHRIFT

(11) **EP 1 671 119 B1**
(45) Veröffentlichungstag und Bekanntmachung des Hinweises auf die Patenterteilung: **30.07.2008**
(21) Anmeldenummer: 04765736.6
(22) Anmeldetag: 01.10.2004
(51) Int. Cl.: G01N 33/36, D06B 23/26, G01N 27/22, G01N 27/24

(54) **VERFAHREN UND VORRICHTUNG ZUR BERWACHUNG EINES FADENS**
METHOD AND DEVICE FOR MONITORING A THREAD
PROCEDE ET DISPOSITIF DE CONTROLE D'UN FIL

(30) Priorität: 04.10.2003 DE 10346599
(43) Veröffentlichungstag der Anmeldung: 21.06.2006
(73) Patentinhaber: Oerlikon Textile GmbH & Co. KG, 42897 Remscheid (DE)
(72) Erfinder: WEIGEND, Helmut, 42477 Radevormwald (DE)
(74) Vertreter: Kahlhöfer, Hermann
(86) Internationale Anmeldenummer: PCT/EP2004/010963
(87) Internationale Veröffentlichungsnummer: WO 2005/033697

(56) Entgegenhaltungen:
- EP-A- 0 918 217
- CH-A- 642 168
- DE-A- 19 839 816
- US-A- 3 757 211
- US-A- 5 844 494

## Beschreibung

Die Erfindung betrifft ein Verfahren zur Überwachung eines Fadens beim Schmelzspinnen und Aufwickeln gemäß dem Oberbegriff des Anspruchs 1 sowie eine Vorrichtung zur Durchführung des Verfahrens gemäß dem Oberbegriff des Anspruchs 9.

Beim Spinnen von mehreren synthetischen Fäden werden die Fäden parallel nebeneinander jeweils durch Zusammenfassen einer Vielzahl von frisch gesponnenen Filamenten gebildet. Hierzu werden die Filamente durch nebeneinander angeordnete Spinndüsen einer Spinneinrichtung extrudiert. Nach Abkühlung der Filamente erfolgt der Zusammenschluss des Filamentbündels durch eine Präparationseinrichtung. Hierbei wird der Zusammenhalt zwischen den Filamenten zu einem Faden durch ein Präparationsmittel erreicht. Nachdem die Fäden die Spinneinrichtung verlassen haben, werden sie zur Behandlung durch eine Behandlungseinrichtung geführt und anschließend jeweils zu einer Spule aufgewickelt. Um die Fäden in gewünschter Weise zu behandeln und aufzuwickeln sowie einen für die Weiterbehandlung gewünschten Zustand der Fäden zu erhalten, ist das Vorhandensein eines Präparationsauftrages an den Fäden erforderlich. Für den Fall, dass ein Faden keinen oder einen unzureichenden Präparationsauftrag aufweist, können einzelne Filamentbrüche bis hin zu einem Fadenbruch entstehen. Um derartige Erscheinungen zu verhindern, ist aus der DE 198 39 816 A1 ein Verfahren und eine Vorrichtung zur Überwachung eines Fadens beim Schmelzspinnen und Aufwickeln-bekannt.

Das aus der DE 198 39 816 A1 bekannte Verfahren basiert darauf, dass zur genauen Bestimmung des Präparationsauftrages der Faden an zwei Messstellen einmal mit und einmal ohne Präparationsauftrag gemessen wird. Aus dem Vergleich der Messsignale lässt sich somit auf die Menge des Präparationsauftrages schließen. Das bekannte Verfahren ist darauf abgestellt, möglichst eine kontinuierliche Schichtdicke an dem Faden zu erhalten. In Praxis werden derartige Fäden jedoch aus einer Vielzahl von einzelnen Filamenten gebildet, die in sich jeweils einen Präparationsauftrag aufweisen müssen. Insofern ist eine äußere Schicht der Präparation nicht unbedingt festzustellen. Es lassen sich somit keine exakten Schichtdickenmessungen ausführen.

Demgemäß ist es Aufgabe der Erfindung ein Verfahren und eine Vorrichtung der gattungsgemäßen Art derart weiterzubilden, dass vorgenannte Nachteile vermieden werden und gleichzeitig eine die praktischen Belange beim Schmelzspinnen und Aufwickeln berücksichtigende sichere Prozessführung erreicht wird.

Diese Aufgabe wird erfindungsgemäß durch ein Verfahren mit den Merkmalen gemäß Anspruch 1 sowie durch eine Vorrichtung mit den Merkmalen gemäß Anspruch 9 gelöst.

Vorteilhafte Weiterbildungen der Erfindung sind durch die Merkmale und Merkmalskombinationen der jeweiligen Unteransprüche definiert.

Die Erfindung zeichnet sich dadurch aus, dass bei unzureichender Präparation Ursache und Wirkung kombiniert sind. So können die auf einen unzureichenden Präparationsauftrag zurückgehenden Fadenbrüche auf einfache Art und Weise erkannt werden. Umgekehrt lassen sich alle nicht mit dem Präparationsauftrag im Zusammenhang stehende Fadenbrüche identifizieren. Hierzu wird erfindungsgemäß die durch die Ladung eines Fadens verursacht Kapazitätsänderung einerseits zur Bestimmung eines Fadenbruchs und andererseits zur Kontrolle eines Präparationsauftrages des Faden ausgewertet. Hierbei lässt sich tendenziell feststellen, ob beispielsweise eine Mindestmenge an Präparation in dem Faden enthalten ist. So ist es bekannt, dass die elektrische Ladung des Fadens wesentlich durch den Präparationsauftrag beeinflusst wird. So würde beispielsweise ein trockener Faden im Vergleich zu einem präparierten Faden zu einer stärkeren Kapazitätsänderung führen. Aus der Intensität eines Messsignals lässt sich somit auf den Präparationszustand des Fadens schließen. Demgegenüber gibt das Vorhandensein einer Kapazitätsänderung Auskunft darüber, ob ein Faden sensiert wird oder ob im Fall eines Fadenbruchs kein Faden sensiert wird. Durch die erfindungsgemäße Kombination lässt sich ein Schmelzspinnprozess auf einfache Art und Weise und mit einfachen Mitteln auf Fadenbruch und Fadenpräparierung überwachen.

Das durch die Kapazitätsänderung ausgelöste Messsignal wird zur Bestimmung eines Fadenbruchs mit einem Schwellenwert verglichen, welcher den Zustand des Kondensators mit einem sensierten Faden darstellt. Für den Fall, dass das Messsignal zu einer Unterschreitung des Schwellenwertes führt, wird ein Steuersignal erzeugt.

Zur Kontrolle des Präparationsauftrages des Fadens wird vorgeschlagen, dass das Messsignal mit einem Toleranzbereich verglichen wird und dass bei einer Toleranzbereichüberschreitung ein Steuersignal erzeugt wird.

Das Steuersignal löst dabei vorzugsweise eine Prozessänderung aus.

Die Weiterbildung der Erfindung, bei welcher das Messsignal in ein vielstufiges Digitalsignal und in ein weiteres zweistufiges Digitalsignal umgewandelt wird, ist besonders geeignet, um eine getrennte Auswertung durchzuführen. Hierbei wird das vielstufige Digitalsignal zur Kontrolle des Präparationsauftrages und das zweistufige Digitalsignal zur Bestimmung des Fadenbruches genutzt.

Die Weiterbildung der Erfindung gemäß den Ansprüchen 7 und 13 ist besonders geeignet, um eine Fadenschar in einem Spinnprozess zu überwachen. Hierbei sind die den Fadenführungsmitteln zugeordneten Kondensatoren derart über eine Messelektronik verknüpft, dass die Messsignale einzeln zur Bestimmung des Messelektronik verknüpft, dass die Messsignale einzeln zur Bestimmung des Fadenbruchs und zu Kontrolle des Präparationsauftrages ausgewertet werden.

Bei der Verwendung einer Walzenpräparationseinrichtung lässt sich die Weiterbildung der Erfindung gemäß Anspruch 8 ausführen. Durch Einbaufehler und Schrägstellungen der Walze bzw. des der Walze zugeordneten Präparationsbehälters machen sich Fehlaunräge insbesondere an den im Randbereich der Fadenschar geführten Fäden bemerkbar. Somit reicht eine Überwachung des Präparationsauftrages an den äußeren Fäden der Fadenschar.

Weitere Vorteile der Erfindung werden nachfolgend anhand einiger Ausführungsbeispiele unter Hinweis auf die beigefügten Zeichnungen näher beschrieben.

Es stellen dar:
- Fig. 1: schematisch ein Ausführungsbeispiel der erfindungsgemäßen Vorrichtung zur Durchführung des erfindungsgemäßen Verfahrens
- Fig. 2: schematisch ein weiteres Ausführungsbeispiel der erfindungsgemäßen Vorrichtung
- Fig. 3: schematisch das Ausführungsbeispiel aus Fig. 2 in der Anwendung in einer Spinnvorrichtung

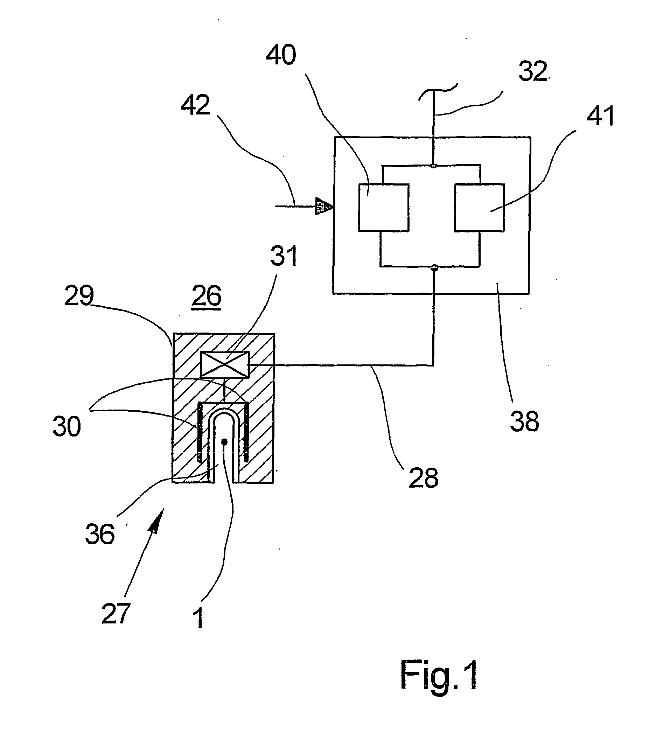

In Fig. 1 ist schematisch ein erstes Ausführungsbeispiel der erfindungsgemäßen Vorrichtung zur Durchführung des erfindungsgemäßen Verfahrens dargestellt. Die Vorrichtung weist einen Messfühler 26 auf. Der Messfühler 26 wird durch ein Gehäuse 29 und ein an dem Gehäuse 29 ausgebildetes Führungsmittel 27 gebildet. Das Führungsmittel 27 ist hierbei durch eine Führungsnut 36 an einer Seite des Gehäuses 29 gebildet. Die Führungsnut 36 weist an ihren Nutwandungen Keramiküberzug auf. Innerhalb des Gehäuses 29 wird die Führungsnut 36 durch einen Kondensator 30 eingeschlossen. Der Kondensator 30 ist mit einer Messelektronik 31 verknüpft. Die Messelektronik 31 ist über eine Signalleitung 28 mit einer Auswerteelektronik 38 verknüpft. Die Auswerteelektronik 38 ist in diesem Fall separat außerhalb des Gehäuses dargestellt. Sie kann jedoch innerhalb des Gehäuses 30 mit der Messelektronik 31 eine gemeinsame Einheit bilden.

Die Signalleitung 28 ist innerhalb der Auswertelektronik 38 auf einer Signaleinlassseite parallel mit einem Komparator 40 und mit einer A/D-Schaltung 41 gekoppelt. Auf der Auslassseite ist die Auswertelektronik 38 über eine Steuerleitung 32 mit einer hier nicht dargestellten Steuereinrichtung verbunden.

Bei der in Fig. 1 dargestellten Vorrichtung wird zur Überwachung eines Schmelzspinn-Aufwickelprozesses ein Faden 1 innerhalb der Führungsnut 36 geführt. Der Faden 1 kann hierbei mit Kontakt oder ohne Kontakt innerhalb der Führungsnut 30 geführt werden. Gleichzeitig wird durch den zuvor aufgeladenen Kondensator 30 ein elektrisches Feld erzeugt. Die in dem Faden 1 mitgeführte elektrische Ladung bewirkt eine Kapazitätsänderung des Kondensators 30, die innerhalb der Messelektronik 31 zu einem Messsignal führt. Das Messsignal, das beispielsweise durch ein Rauschsignal unterschiedlicher Frequenz und Intensität gebildet ist, wird über die Signalleitung 28 der Auswertelektronik 38 zugeführt.

Innerhalb der Auswertelektronik 38 wird zur Bestimmung eines Fadenbruchs das Messsignal zu einem Komparator 40 geleitet. Innerhalb des Komparators 40 erfolgt ein Vergleich mit einem vorgegebenen Schwellenwert. Der Schwellenwert kann über einen zusätzlichen Signaleingang 42 oder in einer Speichereinheit hinterlegt sein. Hierbei kennzeichnet der Schwellenwert den Zustand mit einem Faden. Das Messsignal wird in ein zweistufiges Digitalsignal umgewandelt. Hierbei wird eine erste Stufe des Digitalsignals nur dann erzeugt, wenn der Schwellenwert überschritten wird. Dieser Zustand entspricht dem Vorhandensein eines Fadens, so dass die erste Stufe des Digitalsignals ein Signal "Kein Fadenbruch" erzeugt. Die zweite Stufe des Digitalsignals wird bei Unterschreitung des Schwellenwertes erzeugt. Die zweite Stufe des Digitalsignals signalisiert hierbei einen Fadenbruch, so dass unmittelbar hieraus ein Steuersignal abgeleitet wird und über die Steuerleitung 32 von der Auswertelektronik 38 zu einer nachfolgenden Steuereinrichtung geleitet wird. Innerhalb der Steuereinrichtung wird das Steuersignal eine Prozessänderung auslösen.

Parallel zur Erkennung des Fadenbruchs wird das Messsignal in der A/D-Schaltung 41 ausgewertet Hierzu könnte beispielsweise das analoge Messsignal zunächst verstärkt und gleichgerichtet werden. Nach einer Wandlung in ein vielstufiges Digitalsignal lässt sich pro Zeiteinheit ein Mittelwert ermitteln. Dem Mittelwert wird ein Toleranzbereich zugeordnet. Bei Überschreitung des Toleranzbereiches erfolgt eine Auslösung eines Steuersignals, das über eine Steuerleitung 32 von der Auswertelektronik 38 zu einer Steuereinrichtung geführt wird. Für den Fall, dass der Mittelwert keine Grenzwertüberschreitung kennen lässt, bleibt eine Steuersignalerzeugung aus.

Der Toleranzbereich lässt sich über den Signaleingang 42 vorgeben oder kann unmittelbar vor jedem Prozeßbeginn separat bestimmt werden. Hierzu wird vor jedem Prozeßbeginn in einem ersten Messvorgang eine Auswertung des Messsignals derart vorgenommen, das zu einem ermittelten Mittelwert ein Toleranzband festgelegt wird, welches für nachfolgende Messvorgänge als Grenzwerte gelten.

Unabhängig von der Vorgabe der Grenzwerte wird bei einer Überschreitung der Grenzwerte das erzeugte Steuersignal von der Auswertelektronik 38 zu einer Steuereinrichtung geführt. Innerhalb der Steuereinrichtung lässt sich durch das Steuersignal ein Warnsignal beispielsweise durch ein visuelles oder akustisches Signal auslösen, um einen Eingriff in den Prozess durch eine Bedienperson zu veranlassen. Es ist jedoch auch möglich, dass das Steuersignal unmittelbar zu einem Eingriff in dem Prozess insbesondere in die Präparationseinrichtung führt. einem Eingriff in dem Prozess insbesondere in die Präparationseinrichtung führt.

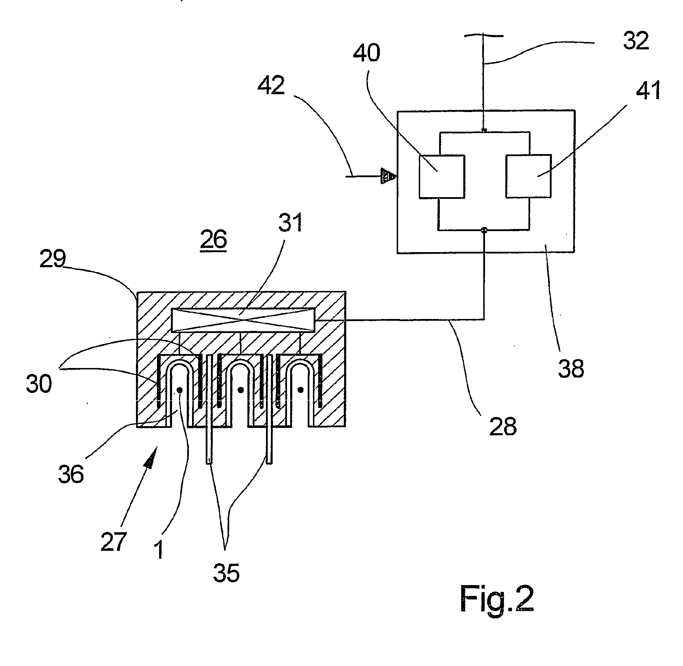

In Fig. 2 ist ein weiteres Ausführungsbeispiel einer erfindungsgemäßen Vorrichtung zur Durchführung des erfindungsgemäßen Verfahrens gezeigt. Der Aufbau und die Funktion des Ausführungsbeispiels nach Fig. 2 ist im wesentlichen identisch zu dem Ausführungsbeispiel nach Fig. 1, so dass auf die vorhergehende Beschreibung Bezug genommen wird und an dieser Stelle nur die Unterschiede erläutert werden.

Bei dem in Fig. 2 dargestellten Messfühler 26 werden insgesamt drei Fäden 1 parallel nebeneinander durch drei parallel nebeneinander ausgebildete Führungsnuten 36 geführt. Hierbei werden in den zugeordneten Kondensatoren 30 Lade- und Entladevorgänge aktiviert, die zu einer jeweiligen Kapazitätsveränderung der Kondensatoren 30 führt. Die Kondensatoren 30 sind mit der Messelektronik 31 verknüpft. Hierbei weist die Messelektronik 31 Mittel auf, um die Messsignale seriell über die Signalleitung 28 der Auswertelektronik 38 zuzuführen.

In dem Gehäuse 29 sind die benachbarten Kondensatoren 30 durch jeweils eine Abschirmplatte 35 voneinander abgeschirmt. Die Abschirmplatte 35 erstreckt sich dabei über die Führungsnut 36 hinaus, so dass die Führungsmittel 27 separiert sind.

Innerhalb der Führungsnuten 36 sind die Nutwände vorzugsweise mit Keramikeinsätzen versehen, so dass selbst bei einer Kontaktführung kein unzulässiger Verschleiß in den Führungsnuten 36 auftritt.

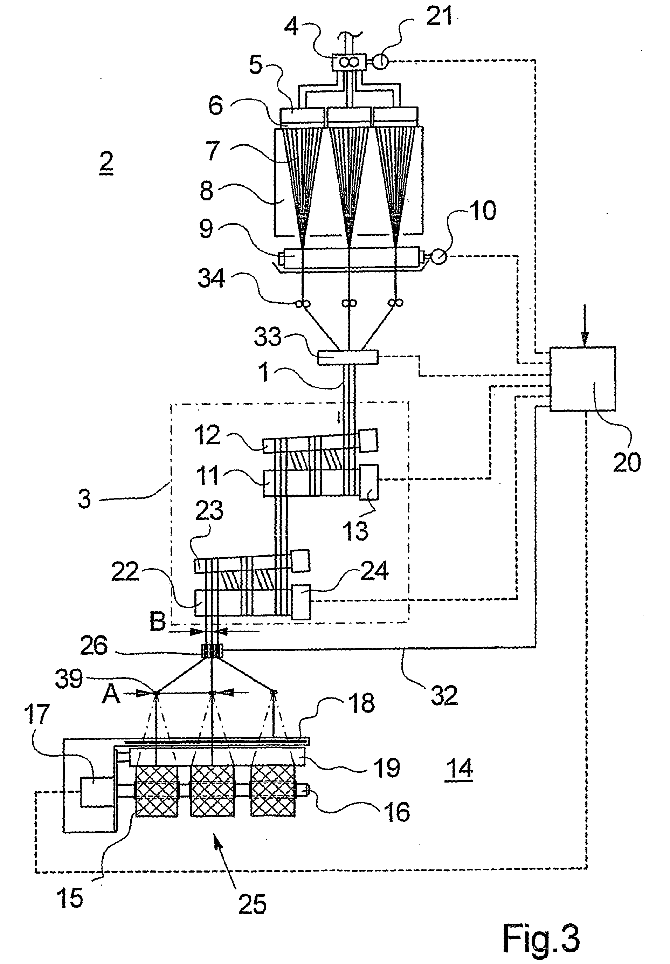

Zur weiteren Erläuterung wird die Anwendung des Ausführungsbeispiels aus Fig. 2 in einer Spinnvorrichtung nachfolgend aufgezeigt. In Fig. 3 ist hierzu ein Ausführungsbeispiel einer Spinnvorrichtung gezeigt.

Die Spinnvorrichtung besteht aus einer Spinneinrichtung 2, eine unterhalb der Spinneinrichtung 2 angeordnete Behandlungseinrichtung 3 sowie eine der Behandlungseinrichtung 3 nachgeordnete Aufwickeleinrichtung 14. Die Spinneinrichtung 2 ist beispielhaft mit drei Spinnstellen ausgeführt, um drei synthetische Fäden parallel nebeneinander zu Spinnen. Hierzu weist die Spinneinrichtung 2 eine Spinnpumpe 4 auf, die beispielsweise als Zahnradpumpe ausgeführt sein kann. Die Spinnpumpe 4 wird durch den Pumpenantrieb 21 angetrieben. Der Pumpenantrieb 21 ist mit einer Steuereinrichtung 20 gekoppelt. Die Spinnpumpe 4 ist als eine Mehrfachpumpe ausgebildet und versorgt parallel mehrere Spinndüsen 5 gleichzeitig. Jede der Spinndüsen 5 besitzt auf der Unterseite eine Düsenplatte 6, durch welche eine Vielzahl von Filamentstränge 7 extrudiert werden. Die Filamentstränge 7 durchlaufen unmittelbar nach dem Extrudieren einen unterhalb der Spinndüsen 5 angeordneten Kühlschacht 8. Innerhalb des Kühlschachtes 8 wird ein Kühlmedium vorzugsweise eine eingeblasene Kühlluft auf die Filamentstränge 7 gerichtet, so daß eine Abkühlung der Filamentstränge 7 innerhalb des Kühlschachtes 8 stattfindet. Am Ende des Kühlschachtes 8 werden die Filamentstränge 7 durch eine Präparationseinrichtung 9 und den Fadenführern 34 zu jeweils einem Faden 1 zusammengeführt. Die Präparationseinrichtung 9 ist als eine Walzenpräparation ausgebildet, bei welcher die Präparationswalze durch einen Walzenantrieb 10 angetrieben wird, so daß die zum Teil in einem Flüssigkeitsbad eingetauchte Präparationswalze gleichmäßig rotiert und die an ihrer Oberfläche geführten Fäden 1 mit einem in dem Bad enthaltenen Präparationsmittel benetzt.

Nachdem die Filamentstränge 7 jeweils zu einem Faden 1 zusammengeführt sind, erfolgt eine weitere Behandlung der Fäden in der nachgeordneten Behandlungseinrichtung. Die Behandlungseinrichtung 3 besteht aus einer Abzugsgalette 11 und einer Streckgalette 22. Der Abzugsgalette 11 ist eine frei drehbare Überlaufrolle 12 und der Streckgalette 22 die frei drehbare Überlaufrolle 23 zugeordnet, so dass die Fäden 1 parallel in mehreren Umschlingungen über die Galetten 11 und 22 geführt werden. Die Abzugsgalette 11 wird über den Galettenantrieb 13 und die Streckgalette 22 durch den Galettenantrieb 24 angetrieben. Die Steuerung der Galettenantriebe 13 und 24 erfolgt durch die Steuereinrichtung 20. Hierbei ist zwischen der Abzugsgalette 11 und der Streckgalette 22 eine Differenzgeschwindigkeit eingestellt, um die Fäden zu verstrecken.

Unterhalb der Behandlungseinrichtung 3 ist die Aufwickeleinrichtung 14 angeordnet, durch welche die Fäden 1 nach der Behandlung zu jeweils einer Spule 15 aufgewickelt werden. Hierzu weist die Wickeleinrichtung 14 insgesamt drei nebeneinander angeordnete Wickelstellen 25 auf. Hierzu sind die Spulen 15 in den Wickelstellen 25 an einer auskragend angetriebenen Spindel 16 gehalten. Die Spindel 16 wird über einen Wickelantrieb 17 derart angetrieben, dass die Fäden 1 mit konstanter Aufwickelgeschwindigkeit zu der Spule 15 aufgewickelt werden. Der Wickelantrieb 17 ist mit der Steuereinrichtung 20 verbunden. Am Umfang der Spule 15 liegt eine von den Fäden teilumschlungene Andrückwalze 19 an. Um die Fäden über die Länge der jeweiligen Spulen 15 zu verteilen, dient eine der Andrückwalze 19 vorgeschaltete Changiereinrichtung 18, die pro Wickelstelle Changierfadenführer aufweist, die die Fäden innerhalb eines Changierhubes hin und herführen.

Zur Prozessüberwachung bei der Herstellung der Fäden 1 ist im Ablaufbereich der Streckgalette 22 ein Messfühler 26 mit mehreren integrierten Fadenführungsmittel 27 angeordnet. Der Aufbau des Messfühlers 26 ist identisch zu dem Ausführungsbeispiel aus Fig. 2. Über eine Steuerleitung 32 ist der Messfühler 26 mit der Steuereinrichtung 20 gekoppelt.

Zwischen der Spinneinrichtung 1 und der Behandlungseinrichtung 3 ist eine Fadensammeleinrichtung 33 in dem Fadenlauf angeordnet, die über die Steuereinrichtung 20 ansteuerbar ist. Die Fadensammeleinrichtung 33 weist Mittel zum Einsammeln, Schneiden und Abführen der Fadenschar auf. Die Fadensammeleinrichtung ist aus der EP 1 049 823 bekannt, so dass an dieser Stelle auf die zitierte Druckschrift Bezug genommen werden kann.

Bei der in Fig. 3 gezeigten Situation werden die Fäden 1 von dem Behandlungsabstand B auf den Spulstellenabstand A aufgespreizt. Hierbei wird die zur Aufspreizung der Fäden 1 erforderliche Fadenführung unmittelbar durch die Messfühler 26 ausgeführt. Der Spulstellenabstand A ist abhängig von der Spulenbreite und liegt im Bereich von 85 bis 250 mm. Hierzu ist jeder Spulstelle 25 ein Kopffadenführer 39 vorgeordnet, so dass die Aufspreizung der Fäden 1 von dem Behandlungsabstand B zu dem Spulstellenabstand A durch den Messfühler 26 und die Kopffadenführer 39 bewirkt wird.

Für den Fall, dass bei der in Fig. 3 dargestellten Vorrichtung einer der Fäden 1 einen Fadenbruch erleidet oder einer der Fäden einen zu geringen Präparationsauftrag aufweist, wird dies durch den Messfühler 26 erfasst und über die Steuerleitung der Steuereinrichtung 20 signalisiert. Innerhalb der Steuereinrichtung 20 wird das Steuersignal des Messfühlers 26 bei Fadenbruch in einen Steuerbefehl für die Fadensammeleinrichtung 33 umgewandelt. Die Fadensammeleinrichtung 33 bündelt und durchtrennt die Fäden und führt sie über eine Absaugung zu einem Garnbehälter. Gleichzeitig wird der Pumpenantrieb 21 durch die Steuereinrichtung 20 in eine Kriechgeschwindigkeit umgeschaltet. Die Kriechgeschwindigkeit der Spinnpumpe 4 ist dabei derart gewählt, dass ein Mindestdurchsatz in den Spinndüsen 5 erhalten bleibt. Der Vollständigkeit halber sei noch gesagt, dass die schmelzeführenden Teile durch Heizeinrichtungen kontinuierlich beheizt sind.

In dem Fall, dass der Messfühler 26 an einem der Fäden 1 einen zu geringen Präparationsauftrag signalisiert, wird in der Steuereinrichtung 20 ein Warnsignal ausgelöst. Daraufhin ist für eine Bedienperson angezeigt, dass die Präparationseinrichtung nicht einwandfrei läuft. Es ist jedoch auch möglich, beispielsweise für den Fall, dass an allen Fäden eine unzureichende Präparationsmenge festgestellt wird, innerhalb der Steuereinrichtung 20 das Steuersignal unmittelbar zur Ansteuerung des Walzenantriebes 10 dient, um durch Erhöhung der Umfangsgeschwindigkeit der Präparationswalze 9 den Präparationsauftrag an den Fäden 1 zu erhöhen.

Bei dem in Fig. 3 gezeigten Ausführungsbeispiel der Spinnvorrichtung ist die Vorrichtung zur Überwachung unmittelbar am Ausgang der Behandlungseinrichtung 3 angeordnet. Dieser Anbringungsort sowie der Aufbau der Spinnvorrichtung ist beispielhaft. Grundsätzlich lässt sich eine Vorrichtung gemäß dem Ausführungsbeispiel nach Fig. 1 verwenden, die vorzugsweise unmittelbar einer Wickelstelle der Aufspuleinrichtung zugeordnet sind. Ebenso ist die Ausbildung der Behandlungseinrichtung beispielhaft. Die Behandlungseinrichtung ist im wesentlichen durch die Art des herzustellenden Fadens bestimmt. So lässt sich die Vorrichtung zur Überwachung eines Fadens beim Schmelzspinnen und Aufwickeln in Spinnvorrichtungen zu Herstellung von POY-, FDY- oder HOY-Fäden einsetzen.

### Bezugszeichenliste

- 1: Faden
- 2: Spinneinrichtung
- 3: Behandlungseinrichtung
- 4: Spinnpumpe
- 5: Spinndüse
- 6: Düsenplatte
- 7: Filamentstränge
- 8: Kühlschacht
- 9: Präparationsrolle
- 10: Walzenantrieb
- 11: Abzugsgalette
- 12: Überlaufrolle
- 13: Galettenantrieb
- 14: Aufwickeleinrichtung
- 15: Spule
- 16: Spindel
- 17: Wickelantrieb
- 18: Changierung
- 19: Andrückwalze
- 20: Steuereinrichtung
- 21: Pumpenantrieb
- 22: Streckgalette
- 23: Überlaufrolle
- 24: Galettenantrieb
- 25: Wickelstelle
- 26: Messfühler
- 27: Führungsmittel
- 28: Signalleitung
- 29: Gehäuse
- 30: Kondensator
- 31: Messelektronik
- 32: Steuerleitung
- 33: Fadensammeleinrichtung
- 34: Fadenführer
- 35: Abschirmplatte
- 36: Führungsnut
- 37: Piezoelement
- 38: Auswertelektronik
- 39: Kopffadenführer
- 40: Komparator
- 41: A/D-Schaltung
- 42: Signaleingang

## Patentansprüche

1. Verfahren zur Überwachung eines Fadens (1) beim Schmelzspinnen und Aufwickeln, bei welchem der Faden (1) durch ein elektrisches Feld eines Kondensators (30) geführt und zur Überwachung des Fadens die Kapazität des Kondensators (30) gemessen wird, **dadurch gekennzeichnet, dass** die gemessene Kapazität des Kondensators (30) zur Bestimmung eines Fadenbruchs mit einem Schwellenwert verglichen und zur Kontrolle eines Präparationsauftrages des Fadens mit einem Toleranzbereich verglichen wird.

2. Verfahren nach Anspruch 1, **dadurch gekennzeichnet, dass** bei Unterschreitung des Schwellenwertes ein Steuersignal erzeugt wird.

3. Verfahren nach Anspruch 1 oder 2, **dadurch gekennzeichnet, dass** bei einer Toleranzbereichüberschreitung ein Steuersignal erzeugt wird.

4. Verfahren nach Anspruch 2 oder 3, **dadurch gekennzeichnet, dass** das Steuersignal eine Warnanzeige und/oder eine Prozessänderung auslöst.

5. Verfahren nach einem der vorgenannten Ansprüche, **dadurch gekennzeichnet, dass** das Messsignal in ein vielstufiges Digitalsignal und in ein zweistufiges Digitalsignal umgewandelt wird, wobei das vielstufige Digitalsignal zur Kontrolle des Präparationsauftrages und das zweistufige Digitalsignal zur Bestimmung des Fadenbruchs genutzt wird.

6. Verfahren nach Anspruch 5, **dadurch gekennzeichnet, dass** zur Auswertung des vielstufigen Digitalsignals ein Mittelwert ermittelt wird, welcher mit dem Toleranzbereich verglichen wird.

7. Verfahren nach einem der vorgenannten Ansprüche, **dadurch gekennzeichnet, dass** mehrere eine Fadenschar bildende parallel laufende Fäden gleichzeitig überwacht werden und dass die Messsignale einzeln ausgewertet werden.

8. Verfahren nach Anspruch 7, **dadurch gekennzeichnet, dass** die Messsignale der im mittleren Bereich der Fadenschar laufenden Fäden zur Bestimmung eines Fadenbruchs ausgewertet werden und dass die übrigen Messsignale zur Bestimmung eines Fadenbruchs und zur Überwachung des Präparationsauftrages ausgewertet werden.

9. Vorrichtung zur Durchführung des Verfahrens nach einem der Ansprüche 1 bis 8, mit einem Führungsmittel (27) zur Führung des Fadens, mit einem Kondensator (30), welcher dem Führungsmittel (27) derart zugeordnet ist, dass der Faden (1) durch ein elektrisches Feld des Kondensators (30) geführt wird, und mit einer Messelektronik (31) zur Erzeugung eines Messsignals, **dadurch gekennzeichnet, dass** eine mit der Messelektronik (31) verbundene Auswertungselektronik (38) vorgesehen ist, die so eingerichtet ist, dass sie das Messsignal zur Bestimmung eines Fadenbruchs mit einem Schwellenwert und zur Kontrolle eines Präparationsauftrages des Fadens (1) mit einem Toleranzbereich vergleicht.

10. Vorrichtung nach Anspruch 9, **dadurch gekennzeichnet, dass** die Auswertelektronik (38) einen Komparator (40) zum Vergleich des Messsignals mit dem Schwellenwert aufweist.

11. Vorrichtung nach Anspruch 9 oder 10, **dadurch gekennzeichnet, dass** die Auswertelektronik (38) eine A/D-Schaltung (41) zum Vergleich des Messsignals mit dem Toleranzbereich aufweist.

12. Vorrichtung nach einem der Ansprüche 9 bis 11, **dadurch gekennzeichnet, dass** die Auswertelektronik (38) mit einer Steuereinrichtung (20) verbunden ist, durch welche Aggregate (21, 10, 33) zum Spinnen und Aufwickeln des Fadens (1) steuerbar sind.

13. Vorrichtung nach einem der Ansprüche 9 bis 12, **dadurch gekennzeichnet, dass** mehrere Fadenführungsmittel (27) an einem Gehäuse (29) ausgebildet sind, dass die den Fadenführungsmittel (27) zugeordneten Kondensatoren (30) elektrisch miteinander verknüpft und gemeinsam an einer Messelektronik (31) angeschlossen sind.

14. Vorrichtung nach Anspruch 13, **dadurch gekennzeichnet, dass** die elektrische Verknüpfung der Kondensatoren (30) in der Messelektronik (31) derart ausgebildet ist, dass die Messsignalen einzeln der Auswertelektronik (38) zuführbar sind.

## Claims

1. Method for monitoring a thread (1) during melt spinning and winding up, in which the thread (1) is guided through an electric field of a capacitor (30) and in which the capacitance of the capacitor (30) is measured in order to monitor the thread, **characterised in that** the capacitance of the capacitor (30) measured is compared to a threshold value in order to determine a thread breakage and to control a spin finish application of the thread is compared to a tolerance region.

2. Method according to claim 1, **characterised in that** a control signal is generated if the threshold value is not reached.

3. Method according to claim 1 or 2, **characterised in that** a control signal is generated if the tolerance region is exceeded.

4. Method according to claim 2 or 3, **characterised in that** the control signal triggers a warning and/or initiates a process change.

5. Method according to one of the preceding claims, **characterised in that** the measuring signal is converted into a multi-stage digital signal and into a two-stage digital signal, wherein the multi-stage digital signal is used to check the spin finish application and the two-stage digital signal to determine the thread breakage.

6. Method according to claim 5, **characterised in that** a mean value, which is compared with the tolerance region, is determined in order to evaluate the multi-stage digital signal.

7. Method according to one of the preceding claims, **characterised in that** a plurality of threads forming a group of threads and running in parallel are monitored at the same time and that the measuring signals are evaluated individually.

8. Method according to claim 7, **characterised in that** the measuring signals of the threads running in the central region of the group of threads are evaluated in order to determine a thread breakage, and that the other measuring signals are evaluated in order to determine a thread breakage and to monitor the spin finish application.

9. Device for carrying out the method according to any one of claims 1 to 8, with a guide means (27) for guiding the thread, with a capacitor (30) which is associated with the guide means (27) such that the thread (1) is guided through an electric field of the capacitor (30), and with an electronic measuring system (31) for generating a measuring signal, **characterised in that** an electronic evaluation system (38) is provided, which is arranged **in that** way that it compares the measuring signal for the determination of a thread breakage with a threshold value and in order to monitor a spin finish application of the thread (1) with a tolerance region.

10. Device according to claim 9, **characterised in that** the electronic evaluation system (38) comprises a comparator (40) for comparing the measuring signal with the threshold value.

11. Device according to claim 9 or 10, **characterised in that** the electronic evaluation system (38) comprises an A/D circuit (41) for comparing the measuring signal with the tolerance region.

12. Device according to any one of claims 9 to 11, **characterised in that** the electronic evaluation system (38) is connected to a control unit (20) by means of which units (21, 10, 33) for spinning and winding up the thread (1) can be controlled.

13. Device according to any one of claims 9 to 12, **characterised in that** a plurality of thread guide means (27) are formed at a housing (29), that the capacitors (30) associated with the thread guide means (27) are electrically interlinked and jointly connected to an electronic measuring system (31).

14. Device according to claim 13, **characterised in that** the electrical linkage of the capacitors (30) in the electronic measuring system(31) is formed such that the measuring signals can be fed individually to the electronic evaluation system (38).

## Revendications

1. Procédé pour surveiller un fil (1) durant le filage à l'état fondu et l'enroulement, dans lequel le fil (1) est guidé à travers un champ électrique d'un condensateur (30) et pour surveiller fil la capacité du condensateur (30) est mesurée, **caractérisé en ce que** pour déterminer une rupture du fil la capacité mesurée du condensateur (30) est comparée à une valeur seuil et est comparée à une plage de tolérance pour le contrôle d'un enduit d'ensimage du fil.

2. Procédé selon la revendication 1, **caractérisé en ce que** quand la valeur seuil n'est pas atteinte un signal de commande est généré.

3. Procédé selon la revendication 1 ou 2, **caractérisé en ce que** quand la plage de tolérance est dépassée, un signal de commande est généré.

4. Procédé selon la revendication 2 ou 3, **caractérisé en ce que** le signal de commande déclenche un voyant d'avertissement et/ou une modification du processus.

5. Procédé selon l'une des revendications précédentes, **caractérisé en ce que** le signal de mesure est transformé en un signal numérique à étages multiples et en un signal numérique biétagé, le signal numérique à étages multiples étant utilisé pour le contrôle de l'enduit d'ensimage et le signal numérique biétagé pour déterminer la rupture du fil.

6. Procédé selon la revendication 5, **caractérisé en ce qu'**une valeur moyenne est déterminée pour l'évaluation du signal numérique à étages multiples, laquelle valeur moyenne est comparée à la plage de tolérance.

7. Procédé selon l'une des revendications précédentes, **caractérisé en ce qu'**une pluralité de fils formant une portée de fils et avançant parallèlement est surveillée en même temps et **en ce que** les signaux de mesure sont évalués individuellement.

8. Procédé selon la revendication 7, **caractérisé en ce que** les signaux de mesure des fils avançant dans la région centrale de la portée de fils sont évalués pour déterminer une rupture de fil et **en ce que** les autres signaux de mesure sont évalués pour déterminer une rupture de fil et pour surveiller l'enduit d'ensimage.

9. Dispositif pour la mise en oeuvre du procédé selon l'une des revendications 1 à 8, avec un moyen de guidage (27) pour guider le fil, avec un condensateur (30) qui est associé de manière telle au moyen de guidage (27), que le fil (1) est guidé à travers un champ électrique du condensateur (30), et avec une électronique de mesure (31) pour générer un signal de mesure, **caractérisé en ce qu'**une électronique d'évaluation (38) est prévue qui est liée à l'électronique de mesure (31), l'électronique d'évaluation (38) étant installée de façon telle qu'elle compare le signal de mesure pour la détermination d'une rupture de fil avec une valeur seuil et avec une plage de tolérance pour le contrôle d'un enduit d'ensimage du fil (1).

10. Dispositif selon la revendication 9, **caractérisé en ce que** l'électronique d'évaluation (38) est dotée d'un comparateur (40) pour comparer le signal de mesure avec la valeur seuil.

11. Dispositif selon la revendication 9 ou 10, **caractérisé en ce que** l'électronique d'évaluation (38) a un circuit A/D (41) pour comparer le signal de mesure à la plage de tolérance.

12. Dispositif selon l'une des revendications 9 à 11, **caractérisé en ce que** l'électronique d'évaluation (38) est en connexion avec une installation de commande (20), par laquelle des agrégats (21, 10, 33) pour filer et pour enrouler le fil (1) peuvent être commandés.

13. Dispositif selon l'une des revendications 9 à 12, **caractérisé en ce qu'**une pluralité de moyens de guidage de fil (27) est formée sur un boîtier (29) que les condensateurs (30) associés aux moyens de guidage de fil (27) sont liés électriquement entre eux et sont raccordés communément à une électronique de mesure (31).

14. Dispositif selon la revendication 13, **caractérisé en ce que** la liaison électrique des condensateurs (30) est réalisée de manière telle dans l'électronique de mesure (31) que les signaux de mesure peuvent être amenés individuellement à l'électronique d'évaluation (38).
